(19) Europäisches Patentamt
European Patent Office
Office européen des brevets

(11) Veröffentlichungsnummer: **0 002 806**
**B1**

(12) # EUROPÄISCHE PATENTSCHRIFT

(45) Veröffentlichungstag der Patentschrift: **29.04.81**

(51) Int. Cl.³: **C 07 C 131/08**

(21) Anmeldenummer: **78101800.7**

(22) Anmeldetag: **21.12.78**

(54) **Verfahren zur Herstellung von in 1-Stellung verätherten 1-Hydroxy-oximino-cycloalkenen-(1).**

(30) Priorität: **04.01.78 DE 2800212**

(43) Veröffentlichungstag der Anmeldung:
**11.07.79 Patentblatt 79/14**

(45) Bekanntmachung des Hinweises auf die Patenterteilung:
**29.04.81 Patentblatt 81/17**

(84) Benannte Vertragsstaaten:
**BE CH DE FR GB**

(56) Entgegenhaltungen:
**DE - A - 2 523 188**
**US - A - 3 991 113**
**HOUBEN-WEYL "Methoden der organischen Chemie"**
**4 Auflage, Band X, Teil 4, 1968,**
**GEORG THIEME VERLAG, Stuttgart**

**PROCEEDINGS OF THE CHEMICAL SOCIETY, Januar 1962**
**Letchworth, Herts, Großbritannien**
**G. BADDELEY et al. "The Reactions of Hydroxylamine and its O-Methyl Derivative with Carvone"**

(73) Patentinhaber: **BASF Aktiengesellschaft**
**Carl-Bosch-Strasse 38**
**D-6700 Ludwigshafen (DE)**

(72) Erfinder: **Weitz, Hans-Martin, Dr.**
**Auf dem Koeppel 40**
**D-6702 Bad Duerkheim (DE)**
Erfinder: **Fischer, Rolf, Dr.**
**Bergstrasse 98**
**D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 806 B1

Verfahren zur Herstellung von in 1-Stellung verätherten 1-Hydroxy-oximino-cycloalkenen-(1)

Die Erfindung betrifft ein neues Verfahren zur Herstellung von in 1-Stellung verätherten 1-Hydroxy-oximino-cycloalkenen-(1) durch Umsetzung von in 1-Stellung verätherten Hydroxyoxocycloalkenen mit Hydroxylamin.

Es ist aus Houben-Weyl, Methoden der Organischen Chemie, Band 10/4, Seite 82 (1968) bekannt, daß 3-Oxo-1,5-dimethyl-cyclohexen-(1) bei der Umsetzung mit Hydroxylamin ein $\beta$-Hydroxylamino-oxim, nämlich das 1-Hydroxylamino-5-oximino-1,3-dimethyl-cyclohexan, ergibt.

Aus Proceedings of the Chemical Society 1962, Seite 145 ist weiterhin bekannt, daß substituierte 1-Methyl-6-oxo-cyclohexene-(1), z.B. Carvon, bei der Umsetzung mit Hydroxylamin in substituierte 2-Methyl-3-hydroxylamino-cyclohexanone, z.B. 2-Methyl-3-hydroxylamino-5-isopropenyl-cyclohexanon, übergehen.

1-Alkoxy-oximino-cycloalkene-(1) lassen sich nach der US-Patentschrift 3 991 113 auch als Cyclohexanon, Nitro-sylchlorid und Alkoholen in Gegenwart von Salzsäure in flüssigem Schwefeldioxid als Lösungsmittel herstellen.

Aus den zunäcst entstehenden zersetzlichen Hydrochloriden muß das 1-Alkoxy-6-oximino-cycloalken-(1) durch Neutralisation mit Natriumbicarbonat freigesetzt werden.

Die deutsche Offenlegungsschrift 2 523 188 beschreibt die Herstellung von 1-Alkoxy-oximino-cycloalkenen-(1) durch Umsetzung von in 1-Stellung durch Amine substituierten Oximino-cycloalkenen-(1) mit Alkoholen und Halogenwasserstoff.

Es wurde gefunden, daß man in 1-Stellung verätherte Hydroxy-oximino-cycloalkene-(1) der Formel

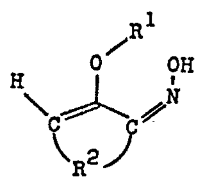

I,

in der $R^1$ einen aliphatischen, cycloaliphatischen, araliphatischen oder aromatischen Rest und $R^2$ einen aliphatischen Rest bedeuten, vorteilhaft erhält, wenn man in 1-Stellung verätherte Hydroxy-oxo-cycloalkene-(1) der Formel

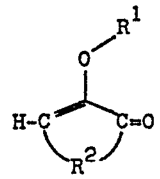

II,

in der $R^1$ und $R^2$ die vorgenannte Bedeutung besitzen, mit 1 bis 3 Mol Hydroxylamin je Mol Ausgangsstoff II umsetzt.

Die Umsetzung wird für den Fall der Verwendung von 1-Äthoxy-6-oxo-cyclohexen-(1) durch die folgenden Formeln wiedergegeben:

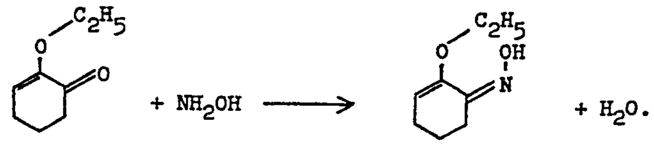


III

Das verfahren nach der Erfindung liefert im Hinblick auf den Stand der Technik auf einfacherem und wirtschaftlicherem Wege in 1-Stellung verätherte Hydroxy-oximino-cycloalkene-(1) in guter Ausbeute, Reinheit sowie Raum-Zeit-Ausbeute. Vorteilhaft gegenüber den beiden bekannten Verfahren ist, daß keine Hydrochloride wie 1-Alkoxy-6-oximino-cycloalken-(1)-hydrochloride oder Amin-hydrochloride, z.B. Morpholin-hydrochlorid, auftreten, aus denen die Salzsäure durch Neutralisation mit Basen entfernt werden muß.

Als Ausgangsstoffe verwendete, in 1-Stellung verätherte Hydroxy-6-oxo-cycloalkene-(1) lassen sich durch Umsetzung des dimeren Halbhydrates des Cyclohexandions-(1,2) mit dem betreffenden Alkohol in Gegenwart von sauren Ionenaustauscherharzen (Chemische Berichte, Band 96, Seiten 826 und 827 (1963)) oder durch Umsetzung von 2,3-Epoxycyclohexanon mit Natriummethylat (Journal of Organic Chemistry, Band 35, Seiten 1 709 bis 1 711 (1970)) herstellen. Der Ausgangsstoff II wird mit 1 bis 3 Mol, vorzugsweise 1,5 bis 2 Mol Hydroxylamin je Mol Ausgangsstoff II umgesetzt. Bevorzugte Ausgangsstoffe II und dementsprechend bevorzugte Endstoffe I sind solche, in deren Formeln $R^1$ einen

Alkylrest mit 1 bis 18, vorzugsweise 1 bis 10 Kohlenstoffatomen, oder einen Halogenalkylrest, vorzugsweise Chloralkylrest, mit 2 bis 18, insbesondere 2 bis 8 Kohlenstoffatomen, einen Cycloalkylrest mit 5 bis 7 Kohlenstoffatomen, einen Aralkylrest mit 7 bis 12 Kohlenstoffatomen oder einen Phenylrest und $R^2$ einen Alkylenrest mit 2 bis 10 Kohlenstoffatomen bezeichnen. Die genannten Rest können noch durch unter den Reaktionsbedingungen inerte Gruppen und/oder Atome, z.B. Alkylgruppen oder Alkoxygruppen mit jeweils 1 bis 4 Kohlenstoffatomen, Hydroxygruppen, Mercaptogruppen, Chloratome, Nitrogruppen, substituiert sein.

Beispielsweise sind folgende in 1-Stellung verätherte 1-Hydroxy-6-oxo-cycloalkene-(1) als Ausgangsstoffe II geeignet: Methyl-, Athyl-, n-Propyl-, Isoproyl-, Butyl-, Isobutyl-, sek.-Butyl-, tert.-Butyl-, Benzyl, Chloräthyl-, Pentyl-, n-Hexyl-, n-Heptyl, n-Octyl-, n-Nonyl-, n-Decyl-, 2-Athylhexyl-, 2,2,5-Trimethyl-n-hexyl-, 2-Athylpentyl-, 3-Athylpentyl-, 2,3-Dimethyl-n-butyl-, 2,2-Dimethyl-n-butyl-, Dodecyl-, Stearyl-, 2-Methylpentyl-, 3-Methylpentyl-, 2,2,4-Trimethylhexyl-, 2-Methylheptyl-, 3-Methylheptyl-, Phenyl-, Cyclohexyl-, Cyclopentyl-, Undecyl-äther-(1) des 1-Hydroxy-6-oxo-cyclohexens-(1); entsprechende in 1-Stellung verätherte 1-Hydroxy-5-oxo-1-cyclopentene und entsprechende Cycloheptene, Cyclooctene, Cyclononene und Cyclodecene.

Die Umsetzung wird im allgemeinen bei einer Temperatur von —20 bis + 150°C, vorzugsweise von +20 bis +100°C, drucklos oder unter Druck, diskontinuierlich oder kontinuierlich durchgeführt. Vorteilhaft verwendet man zusätzlich unter den Reaktionsbedingungen inerte Lösungsmittel, im allgemeinen solche, in denen Hydroxylamin löslich ist. Beispielsweise kommen folgende Alkohole als Lösungsmittel in Betracht: Methyl-, Athyl-, n-Propyl-, Isopropyl-, n-Butyl-, Isobutyl-, sek.-Butyl-, tert.-Butyl-, Pentyl-, n-Hexyl-, n-Heptyl-, n-Octyl-alkohol. Als Lösungsmittel sind insbesondere Wasser und niedere Alkohole wie Methanol, Äthanol, Propanole und Butanole geeignet. Zweckmäßig verwendet man das Lösungsmittel in einer Menge von 10 bis 50 Mol, vorzugsweise 30 bis 50 Mol, bezogen auf 1 Mol Ausgangsstoff II.

Das Hydroxylamin wird in der Regel in Form seiner Salze verwendet. Geeignete Salze sind z.B. das Chlorid, Nitrat, Sulfat, Formiat oder Acetat des Hydroxylamins. Ebenfalls ist es möglich, anstelle der Salze Hydroxylamin selbst dem Reaktionsgemisch zuzuführen. Zweckmäßig stellt man, z.B. in der in Kirk-Othmer, Encyclopedia of Chemical Technology, Second Edition, Band 11, Seite 497 beschriebenen Arbeitsweise, eine Lösung von freiem Hydroxylamin in Wasser und/oder Alkohol her; als Alkohol verwendet man im allgemeinen die vorgenannten, insbesondere Methanol, Äthanol, n-Propanol oder n-Butanol. Man kann auch aus einem Hydroxylammoniumsalze, beispielsweise Hydroxylammoniumchlorid, Hydroxylammoniumsulfat, Hydroxylammoniumnitrat oder Hydroxylammoniumacetat, das in einem der genannten Lösungsmittel gelöst ist, mit Basen wie Natriumhydroxid, Kaliumhydroxid, Calciumhydroxid, Ammoniak, Natriumcarbonat, Natriummethylat oder Natriumäthylat Hydroxylamin freisetzen. Das anfallende Natrium-, Kalium-, Ammonium- oder Calciumsalz wird vor oder nach der Umsetzung mit Ausgangsstoff II abfiltriert.

Die Reaktion kann wie folgt durchgeführt werden: Ein Gemisch von Ausgangsstoff II, Hydroxylamin und in der Regel Lösungsmittel wird bei der Reaktionstemperatur 0,5 bis 2 Stunden gehalten. Die Hydroxylaminlösung wird in der Regel unter Rühren zu einer Lösung des Ausgangsstoffes II in einem der genannten Lösungsmittel hinzugegeben. Es ist auch möglich, ein Gemisch aus Hydroxylammoniumsalz, Base und Ausgangstoff II in einem der genannten Lösungsmittel ohne Herstellung einer Hydroxylaminlösung direkt umzusetzen. Der Endstoff I wird nach der Umsetzung in üblicher Weise, z.B. durch Filtration, gegebenenfalls nach Fällung, beispielsweise mit Wasser, und gegebenenfalls Umkristallisation, abgetrennt.

Die nach dem Verfahren der Erfindung herstellbaren Verbindungen I sind wertvolle Ausgangsstoffe für die Herstellung von Farbstoffen und Schädlingsbekämpfungsmitteln, besitzen fungizide Eigenschaften (US-Patentschrift 3 991 113) und können als Komplexbildner für Schwermetallionen verwendet werden. Sie stellen wichtige Zwischenprodukte für die Synthese der Aminosäure Lysin dar (US-Patentschrift 3 928 445). Durch Reduktion der Doppelbindung unter Erhalt der Oximinogruppe bilden sich verätherte 2-Alkoxy-cycloalkanoxime. Die Beckmannsche Umlagerung dieser in 2-Stellung substituierten Cycloalkanoxime liefert entsprechend substituierte Lactame. Die deutsche Offenlegungsschrift 2 360 406 zeigt die Verwendung von Alkoxylactamen für die Herstellung von Polyamiden und Copolyamiden.

Die in den folgenden Beispielen angeführten Teile bedeuten Gewichtsteile.

## Beispiel 1

6,95 Teile Hydroxylamin-hydrochlorid werden unter Erwärmen in 25 Teilen Methanol gelöst. Hierzu gibt man eine heiße Lösung von 5,6 Teilen KOH in 13 Teilen Methanol. Das beim Abkühlen ausfallende Kaliumchlorid wird abgesaugt. Zu der Hydroxylaminlösung fügt man unter Rühren 8,4 Teile 1-Butoxy-6-oxo-cyclohexen-(1) bei 20 bis 40°C hinzu. Nach 10 Minuten bei 40°C kühlt man das Reaktionsgemisch auf 0°C und filtriert. Man erhält 8,9 Teile 1-Butoxy-6-oximino-cyclohexen-(1) (97% der Theorie) vom Fp 82,5 bis 83,5°C (aus Methanol).

Beispiele 2 bis 7

Analog Beispiel 1 werden weitere 1-Alkoxy-6-oxo-cyclohexene-(1) mit Hydroxylamin umgesetzt. Ausgangsverbindungen, Molverhältnis der Reaktionspartner, Schmelzpunkte und Ausbeuten sind in der folgenden Tabelle enthalten.

TABELLE

| Beispiel | 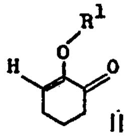 II | Teile Ausgangsstoff II | Teile $NH_2OH \cdot HCl$ | 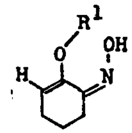 Teile I | Fp °C (umkristallisiert aus) | Ausbeute (% der Theorie) |
|---|---|---|---|---|---|---|
| 2 | $R^1 = -C_2H_5$ | 11,9 | 5,9 | 3,7 | 159–162 (Essigester) | 28 |
| 3 | $R^1 = -C_2H_5$ | 14,0 | 13,9 | 14,3 | 159–162 (Essigester) | 92 |
| 4 | $R^1 = n\text{-}C_3H_7$ | 15,4 | 13,9 | 13,9 | 73–74 ($C_2H_5OH$) | 82 |
| 5 | $R^1 = n\text{-}C_{12}H_{25}$ | 18,4 | 13,9 | 19,2 | 44–45 ($C_2H_5OH$) | 98 |
| 6 | $R^1 = -CH_2-CH_2Cl$ | 9,6 | 7,7 | 7,2 | 107 (Zers.) ($CH_3OH$) | 69 |
| 7 | $R^1 =$ 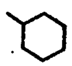 | 43,6 | 31,3 | 39,5 | 133 ($CH_3OH$) | 84 |

**Patentanspruch**

Verfahren zur Herstellung von in 1-Stellung verätherten Hydroxy-oximino-cycloalkenen-(1) der Formel

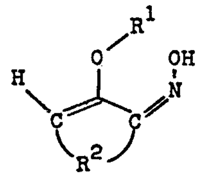

I,

in der $R^1$ einen aliphatischen, cycloaliphatischen, araliphatischen oder aromatischen Rest und $R^2$ einen aliphatischen Rest bedeuten, dadurch gekennzeichnet, daß man in 1-Stellung verätherte Hydroxy-oxo-cycloalkene-(1) der Formel

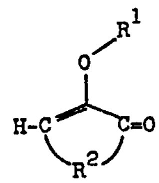

II,

in der $R^1$ und $R^2$ die vorgenannte Bedeutung besitzen, mit 1 bis 3 Mol Hydroxylamin je Mol Ausgangsstoff II umsetzt.

**Claim**

A process for the preparation of a hydroxy-oximino-cycloalk-1-ene, etherified in the 1-position, of the formula

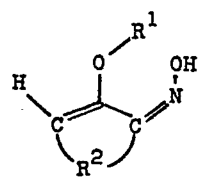

I,

where $R^1$ is an aliphatic, cycloaliphatic, araliphatic or aromatic radical and $R^2$ is an aliphatic radical, characterized in that a hydroxy-oxo-cycloalk-1-ene, etherified in the 1-position, of the formula

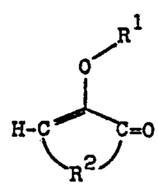

II,

where $R^1$ and $R^2$ have the above meanings, is reacted with from 1 to 3 moles of hydroxylamine per mole of starting material II.

**Revendication**

Procédé de préparation d'hydroxy-oximino-cycloalcènes-(1) éthérifiés en position 1-, de formule

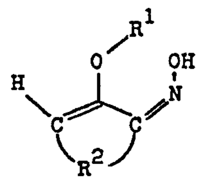

I,

dans laquelle $R^1$ représente un reste aliphatique, cycloaliphatique, araliphatique ou aromatique et $R^2$ un reste aliphatique, caractérisé en ce que l'on fait réagir des hydroxy-oxo-cycloalcènes-(1) éthérifiés en position 1-, répondant à la formule

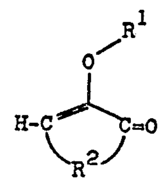

II,

dans laquelle $R^1$ et $R^2$ ont les significations indiquées ci-dessus, avec 1 à 3 moles d'hydroxylamine par mole du composant de départ II.